(19) Europäisches Patentamt
European Patent Office
Office européen des brevets

(11) Veröffentlichungsnummer: **0 442 371 A2**

(12) **EUROPÄISCHE PATENTANMELDUNG**

(21) Anmeldenummer: **91101651.7**

(22) Anmeldetag: **07.02.91**

(51) Int. Cl.5: **C07H 15/04**, C07C 31/20, C11D 3/22, A61K 7/00

(30) Priorität: **16.02.90 DE 4004884**

(43) Veröffentlichungstag der Anmeldung: **21.08.91 Patentblatt 91/34**

(84) Benannte Vertragsstaaten: **CH DE FR GB IT LI**

(71) Anmelder: **BASF Aktiengesellschaft**
**Carl-Bosch-Strasse 38**
**W-6700 Ludwigshafen(DE)**

(72) Erfinder: **Wolf, Gerhard, Dr.**
**Robert-Blum-Strasse 31**
**W-6800 Mannheim 24(DE)**
Erfinder: **Schmidt, Richard R. Prof. Dr.**
**Grossherzog-Friedrich-Strasse 11**
**W-7750 Konstanz 16(DE)**
Erfinder: **Jankowski, Karin**
**Schuetzenstrasse 34**
**W-7750 Konstanz(DE)**
Erfinder: **Terjung, Andreas**
**Schwanenweg 2**
**W-7760 Radolfzell(DE)**

(54) **Bisglykoside.**

(57) Bisglykoside I

$$Gly\text{-}O\text{-}CHR^2\text{-}(CH_2)_p\text{-}(XR^1)_n\text{-}(CH_2)_q\text{-}(CHR^3)_m\text{-}O\text{-}Gly \quad I$$

bei denen die Variablen folgende Bedeutung haben:

| | |
|---|---|
| X | C-$R^4$ oder N |
| $R^1$ | für den Fall X = C-$R^4$: $C_1$-$C_{30}$-Alkyl oder $C_2$-$C_{30}$-Alkenyl oder O-$C_1$-$C_{30}$-Alkyl oder O-$C_2$-$C_{30}$-Alkenyl<br>für den Fall X = N: CO-$C_1$-$C_{30}$-Alkyl oder CO-$C_2$-$C_{30}$-Alkenyl |
| $R^2$ bis $R^4$ | Wasserstoff oder $C_1$-$C_{30}$-Alkyl oder $C_2$-$C_{30}$-Alkenyl |
| n | 1 bis 3 |
| m | 0 oder 1 |
| p,q | 0 bis 2 |
| Gly | aus Aldopentosen, Aldohexosen und/oder Ketohexosen aufgebaute Mono- oder Disaccharide. |

Die Bisglykoside I dienen als oberflächenaktive Substanzen bzw. Emulgatoren in Wasch-, Reinigungs- und Körperpflegemitteln.

EP 0 442 371 A2

Die vorliegende Erfindung betrifft neue Bisglykoside der allgemeinen Formel I

$Gly\text{-}O\text{-}CHR^2\text{-}(CH_2)_p\text{-}(XR^1)_n\text{-}(CH_2)_q\text{-}(CHR^3)_m\text{-}O\text{-}Gly$ I

in der die Variablen folgende Bedeutung haben:

| | |
|---|---|
| X | C-$R^4$ oder N |
| $R^1$ | für den Fall X = C-$R^4$: $C_1$-$C_{30}$-Alkyl oder $C_2$-$C_{30}$-Alkenyl oder O-$C_1$-$C_{30}$-Alkyl oder O-$C_2$-$C_{30}$-Alkenyl<br>für den Fall X = N: CO-$C_1$-$C_{30}$-Alkyl oder CO-$c_2$-$c_{30}$-Alkenyl |
| $R^2$ bis $R^4$ | Wasserstoff oder $C_1$-$C_{30}$-Alkyl oder $C_2$-$C_{30}$-Alkenyl |
| n | 1 bis 3 |
| m | 0 oder 1 |
| p,q | 0 bis 2 |
| Gly | aus Aldopentosen, Aldohexosen und/oder Ketohexosen aufgebaute Mono- oder Disaccharide. |

Weiterhin betrifft die Erfindung ein Verfahren zur Herstellung dieser Bisglykoside, die entsprechenden Diole als Zwischenprodukte für dieses Herstellungsverfahren, die Verwendung der Bisglykoside I als oberflächenaktive Substanzen bzw. Emulgatoren in Wasch-, Reinigungs- und Körperpflegemitteln sowie die Verbindungen I enthaltende Wasch-, Reinigungs- und Körperpflegemittel.

Auf dem Tensid-Sektor sind in den letzten Jahren in zunehmendem Maße nichtionische oberflächenaktive Substanzen auf der Basis nachwachsender Rohstoffe in den Vordergrund getreten. Derartige Stoffe haben in der Regel eine gute biologische Abbaubarkeit, eine geringe Toxizität und somit eine gute Umweltverträglichkeit.

Eine wichtige Gruppe solcher nichtionischer Tenside stellen Alkylglykoside dar, wie sie z.B. in der Literaturstelle Tenside Surf. Det. 26 (1989) 5, 318-324, (1), beschrieben sind, bei denen der langkettige hydrophobe Alkylrest direkt an den hydrophilen Kohlenhydratteil des Moleküls mittels einer acetalischen Bindung angeknüpft ist.

Solche Produkte erfüllen die in sie gesetzten Erwartungen zwar schon weitgehend, erweisen sich aber in einigen Eigenschaften wie dem Solubilisierungsvermögen als noch verbesserungsbedürftig. Erwünscht wäre es außerdem, wenn die oberflächenaktiven Verbindungen in Wasch- und Reinigungsmittel außerdem noch Komplexbildner- und/oder Gerüststoff-Funktionen zumindest teilweise übernehmen könnten.

R.R. Schmidt beschreibt in der Literaturstelle Angew. Chem. 98 (1986), 213-236, (2), die Verwendungsmöglichkeiten von O-Glykosyl-trichloracetimidaten und die Verwendung der Trifluormethansulfonatgruppe als weitere aktivierende Gruppe in der Glykosid- und Oligosaccharid-Synthese.

Der vorliegenden Erfindung lag die Aufgabe zugrunde, nichtionische Tenside auf der Basis nachwachsender Rohstoffe bereitzustellen, die die oben geschilderten Mängel nicht mehr aufweisen.

Demgemäß wurden die eingangs definierten Bisglykoside I gefunden.

Die den Resten $R^1$ bis $R^4$ zugrundeliegenden linearen oder verzweigten Alkyl- oder Alkenylgruppen bestehen aus 1 bis 30 bzw. 2 bis 30 C-Atomen. Besonders gute Ergebnisse liefern Gruppen mit 6 bis 22 C-Atomen, insbesondere 10 bis 18 C-Atomen. Bei einer Kettenlänge von 14 bis 16 C-Atomen wird ein Optimum in den Eigenschaften erreicht, da hierbei offenbar das optimale Größenverhältnis zwischen hydrophobem und hydrophilem Molekülteil vorliegt.

Als Beispiele seien die folgenden Alkyl- bzw. Alkenylgruppen aufgeführt:
Methyl, Ethyl, n-Propyl, n-Butyl, n-Hexyl, n-Octyl, 2-Ethylhexyl, n-Nonyl, iso-Nonyl, n-Decyl, n-Undecyl, n-Dodecyl, n-Tridecyl, iso-Tridecyl, Myristyl, Cetyl, Stearyl, Eisosyl, Vinyl, 1-Propenyl, 2-Propenyl, Oleyl, Linolyl und Linolenyl.

Steht der Rest $R^1$ an einem C-Atom, bezeichnet er eine Alkyl- bzw. Alkenylgruppe oder eine Alkoxy- bzw. Alkenyloxygruppe. Steht der Rest $R^1$ dagegen an einem N-Atom, steht er für eine Alkylcarbonyl- bzw. Alkenylcarbonylgruppe.

Die Reste $R^2$ bis $R^4$ stehen vorzugsweise für Wasserstoff, daneben aber auch für weitere Alkyl- bzw. Alkenylgruppen.

Die Variable n steht vor allem für den Wert 1, daneben aber auch für 2 oder 3. Die Variable m steht für 0 oder 1. Die Variablen p und q stehen unabhängig voneinander für 0, 1 oder 2, insbesondere beide für 0.

Der Glykosidrest Gly bezeichnet Mono- oder Disaccharide, welche aus üblichen Pentosen und/oder Ketosen aufgebaut sind. Als derartige Bausteine dienen Aldopentosen wie Ribose, Arabinose, Xylose und Lyxose, Aldohexosen wie Allose, Altrose, Glucose, Mannose, Gulose, Idose, Galactose und Talose sowie Ketohexosen wie Fructose. Bevorzugt werden hiervon Mannose, Glucose, Galactose und Fructose.

Als Disaccharide können vor allem Saccharose, Lactose, Maltose und Cellobiose verwendet werden.

Je nach den Herstellungsbedingungen der Bisglykoside I können noch geringe Anteile an Oligosacchariden mit einem Glykosidierungsgrad bis 8 und an Polysacchariden mit einem Glykosidierungsgrad größer als 8 als Komponenten in den Verbindungen I enthalten sein.

Eingesetzt werden normalerweise die in der Natur vorkommenden Kohlenhydrate der D-Reihe, es können aber auch Vertreter der L-Reihe verwendet werden.

Die Verknüpfung der Kohlenhydrat-Komponenten mit dem übrigen Molekülteil von I geschieht in der Regel durch eine acetalische Bindung am anomeren C-Atom, d.h. am 1-C-Atom, wobei sowohl $\alpha$- als auch $\beta$-Anomere vorliegen können.

Bevorzugt werden Bisglykoside der allgemeinen Formel Ia

Gly-O-$CHR^2$-$CHR^1$-$(CHR^3)_m$-O-Gly Ia

in der $R^1$ die für den Fall X = C-$R^4$ angegebene Bedeutung hat.

Weiterhin werden bevorzugt Bisglykoside der allgemeinen Formel Ib

Gly-O-$CHR^2$-$(CH_2)_p$-$NR^1(CH_2)_q$-$CHR^3$-O-Gly Ib

in der $R^1$ die für den Fall X = N angegebene Bedeutung hat.

Die Bisglykoside I werden zweckmäßigerweise durch Umsetzung von

(a) Diolen der allgemeinen Formel II

HO-$CHR^2$-$(CH_2)_p$-$(XR^1)_n$-$(CH_2)_q$-$(CHR^3)_m$-OH II

mit geschützten Glykosyl-trihalogenacetimidaten der allgemeinen Formel III

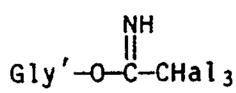

III

in der Hal für Fluor, Chlor oder Brom steht und Gly' einen Glykosylrest von Mono- oder Disacchariden, die aus Aldopentosen, Aldohexosen und/oder Ketohexosen aufgebaut sind und deren übrige Hydroxylgruppen hierbei übliche Schutzgruppen tragen, bezeichnet, oder

(b) aus den Diolen II erhältlichen Bistrifluormethansulfonaten der allgemeinen Formel IV

$CF_3SO_2O$-$CHR^2$-$(CH_2)_p$-$(XR^1)_n$-$(CH_2)_q$-$(CHR^3)_m$-$OSO_2CF_3$ IV

mit geschützten Mono- oder Disacchariden der allgemeinen Formel V

Gly'-OH V

und anschließende Entfernung der Schutzgruppen der Glykosylreste nach den üblichen Methoden hergestellt.

Die den beiden Ausführungsformen (a) und (b) zugrundeliegenden für diese Stoffklassen üblichen Synthesemethoden sind in (2) dargestellt.

Bei den Kohlenhydrat-Verbindungen III und V tragen alle übrigen Hydroxylgruppen außer derjenigen, welche zur Verknüpfung mit dem übrigen Molekülteil von I dient und in der Regel am anomeren C-Atom, d.h. am 1-C-Atom, steht, Schutzgruppen, um unerwünschte Nebenreaktionen auszuschließen.

übliche Schutzgruppen hierfür sind beispielsweise die Acetylgruppe, die Benzylgruppe oder die Isopropylidengruppierung, die sich nach erfolgter Umsetzung wieder leicht nach den üblichen Methoden, beispielsweise mittels katalytischer Mengen eines Natriumalkoholates oder mittels Protonensäuren wie Essigsäure, abspalten lassen.

Bei der Trihalogenacetimidat-Methode (a) werden die Diole II mit den Acetimidaten III normalerweise in Gegenwart katalytischer Mengen einer Protonensäure oder einer Lewis-Säure umgesetzt. Hierfür werden vorzugsweise Lewis-Säuren wie Zinntetrachlorid, Titantetrachlorid und vor allem Bortrifluorid-Addukte, z.B. Bortrifluorid-Etherat, eingesetzt. Von den Acetimidaten III sind am vorteilhaftesten die Trichloracetimidate verwendbar.

Bei Methode (a) arbeitet man in der Regel in einem wasserfreien inerten Lösungsmittel wie n-Pentan, n-Hexan, Cyclohexan, Toluol, Chloroform, Dichlormethan, Tetrahydrofuran oder Diethylether oder in einem Gemisch solcher Lösungsmittel bei Raumtemperatur oder leicht erhöhter Temperatur bis etwa 50° C und bei Normaldruck. Die Umsetzungen sind meist nach 1 bis 5 Stunden beendet. Für eine eventuelle Reinigung des Produktes eignen sich am besten chromatographische Methoden.

Bei der Trifluormethansulfonat-Methode (b) werden die aus den Diolen II beispielsweise durch Umsetzung mit Trifluormethansulfonsäureanhydrid erhältlichen Bistrifluormethansulfonate IV mit den Kohlenhydraten V in der Regel in Gegenwart einer starken Base wie einem Alkalimetallalkoholat oder einem Alkalimetallhydrid umgesetzt. Man arbeitet hierbei normalerweise in einem wasserfreien inerten Lösungsmittel wie bei (a) bei Raumtemperatur oder erhöhter Temperatur bis etwa 80° C und bei Normaldruck. Die Verwendung eines Schutzgases wie Stickstoff oder Argon ist empfehlenswert. Für eine eventuelle Reinigung des Produktes eignen sich am besten chromatographische Methoden.

Gegenstand der vorliegenden Erfindung sind weiterhin Diole der allgemeinen Formel II

$HO\text{-}CHR^2(CH_2)_p\text{-}(XR^1)_n\text{-}(CH^2)_q\text{-}(CHR^3)_m\text{-}OH$ II

als Zwischenprodukt für die Herstellung der Bisglykoside I.

Die Bisglykoside I finden Verwendung als oberflächenaktive Substanzen, hauptsächlich als nichtionische Tenside oder Emulgatoren, in Wasch- und Reinigungsmitteln, beispielsweise für Reinigungsprozesse in Technik und Haushalt wie für die Textilwäsche oder für Reinigungsprozesse im Nahrungsmittelbereich wie die Reinigung von Getränkeflaschen. Weiterhin dienen sie in Körperpflegemitteln wie Hautcremes, Lotionen, Gelen, Hautölen oder Haarshampoos als Emulgatoren.

In Wasch- und Reinigungsmitteln wirken die Bisglykoside I außerdem als Komplexbildner und Gerüststoffe (Builder), was auf die chelatisierende Wirkung der beiden Kohlenhydrat-Einheiten zurückzuführen ist. Durch die Wechselwirkung mit den Hydroxylgruppen der Kohlenhydrat-Gruppierungen werden Metallionen wie Calcium-, Magnesium- und Eisenionen komplexiert, was zu einer Verstärkung der Waschwirkung führt.

Gegenstand der vorliegenden Erfindung sind ebenfalls Wasch-, Reinigungsund Körperpflegemittel, welche 1 bis 50 Gew.-%, vorzugsweise 5 bis 30 Gew.-% eines Bisglykosides I oder einer Mischung solcher Bisglykoside enthalten. Die üblichen Bestandteile und Zusammensetzung von Wasch- und Reinigungsmitteln sowie von Körperpflegemitteln sind dem Fachmann bekannt und brauchen deshalb hier nicht weiter erörtert zu werden.

Die erfindungsgemäßen Bisglykoside I führen zu einer niedrigen Oberflächenspannung im Bereich von etwa 30 bis etwa 50 mN/m und vor allem einer sehr niedrigen kritischen Micellbildungskonzentration (CMC) im Bereich von etwa 1 bis etwa 0,001 mmol/l. Die geringen CMC-Werte sind Ausdruck des ausgezeichneten Solubilisierungsvermögens der Verbindungen I. Daher werden in Wasch-, Reinigungs- und Körperpflegemittelformulierungen wesentlich geringere Konzentrationen der erfindungsgemäßen Verbindungen I als bei üblichen Alkylglykosiden benötigt.

Die biologisch leicht abbaubaren Bisglykoside I weisen außerdem den Vorteil auf, daß sie in Wasch- und Reinigungsmitteln zusätzlich als Komplexbildner und Gerüststoffe wirken und somit übliche schwer biologisch abbaubare Komplexbildner und Gerüststoffe zumindest teilweise ersetzen können, wodurch die Umweltbelastung verringert werden kann.

Herstellungsbeispiele

Beispiel 1

2-(β-D-Glucopyranosyloxymethyl)dodecyl-1-β-D-glucopyranosid

2,05 g (4,18 mmol) 2,3,4,6-Tetra-O-acetyl-α-D-glucopyranosyl-trichloracetimidat und 0,43 g (2,0 mmol) 2-Hydroxymethyl-1-dodecanol wurden in 20 ml einer Mischung aus gleichen Volumenteilen wasserfreiem n-Hexan und wasserfreiem Dichlormethan gelöst. Unter Feuchtigkeitsausschluß wurden langsam 2,0 g einer ca. 1,5 gew.-%igen Lösung von Bortrifluorid-Etherat in Diethylether (entsprechend 0,2 mmol $BF_3 \cdot Et_2O$) zugetropft.

Nach 2,5 Stunden Rühren bei Raumtemperatur wurden 0,3 g Natriumhydrogencarbonat und 20 ml Dichlormethan zugegeben. Nach Filtration, Abdestillieren der Lösungsmittel und Reinigung des erhaltenen Rohproduktes durch Flashchromatographie (Toluol/Essigsäureethylester 7:3) wurde das Produkt in 60 ml wasserfreiem Methanol gelöst und die Acetyl-Schutzgruppen wurden durch 5 stündiges Rühren mit 0,5 ml einer 0,1 molaren Natriummethanolat-Lösung in Methanol bei Raumtemperatur abgespalten.

Nach Behandeln mit saurem Ionenaustauschermaterial wurde die Lösung im Wasserstrahlvakuum

eingeengt und das Endprodukt durch Flashchromatographie (Dichlormethan/Methanol 5:3) gereinigt. Die Titelverbindung wurde in einer Ausbeute von 52 % erhalten und hatte einen Schmelzpunkt von 146 - 146,5 ° C und einen spezifischen Drehwert von $[\alpha]^{20}{}_D$ = -24,7 ° .

Beispiel 2

2-(β-D-Glucopyranosyloxymethyl)-tetradecyl-1-β-D-glucopyranosid

Ausgehend von 2-Hydroxymethyl-1-tetradecanol wurde die Titelverbindung in einer Ausbeute von 53 % analog zu Beispiel 1 hergestellt. Sie hatte einen Schmelzpunkt von 181,1 - 181,6 ° C und einen spezifischen Drehwert von $[\alpha]^{20}{}_D$ = -8,5 ° .

Beispiel 3

2-(β-D-Glucopyranosyloxymethyl)-hexadecyl-1-β-D-glucopyranosid

Ausgehend von 2-Hydroxymethyl-1-hexadecanol wurde die Titelverbindung in einer Ausbeute von 69 % analog zu Beispiel 1 hergestellt. Sie hatte einen Schmelzpunkt von 154,1 - 154,6 ° C und einen spezifischen Drehwert von $[\alpha]^{20}{}_D$ = -18,2 ° .

Beispiel 4

2(β-D-Glucopyranosyloxymethyl)-octadecyl-1-β-D-glucopyranosid

Ausgehend von 2-Hydroxymethyl-1-octadecanol wurde die Titelverbindung in einer Ausbeute von 43 % analog zu Beispiel 1 hergestellt. Sie hatte einen Schmelzpunkt von 214,1 - 214,7 ° C und einen spezifischen Drehwert von $[\alpha]^{20}{}_D$ = -9,7 ° .

Beispiel 5

2-Decyloxy-3-(β-glucopyranosyloxy)-propyl-1-β-D-glucopyranosid

Ausgehend von 2-Decyloxy-1,3-propandiol wurde die Titelverbindung in einer Ausbeute von 62 % analog zu Beispiel 1 hergestellt. Sie hatte einen Schmelzpunkt von 164,5 - 164,8 ° C und einen spezifischen Drehwert von $[\alpha]^{20}{}_D$= -12,9 ° .

Beispiel 6

2-Decyloxy-1,3-di(β-D-mannofuranosyloxy)-propan

3,90 g (15,0 mmol) 2,3:5,6-Di-O-isopropyliden-mannofuranose wurden unter Stickstoff als Schutzgas in 100 ml wasserfreiem Toluol gelöst und mit 400 mg Natriumhydrid versetzt. Nach beendeter Wasserstoffentwicklung wurde auf 50 ° C erhitzt. 3,72 g (7,5 mmol) 2-Decyloxy-1,3-di-(trifluormethansulfonyloxy)-propan wurden langsam zugetropft.

Nach beendeter Reaktion wurde überschüssiges Natriumhydrid unter Kühlung mit Methanol zersetzt, die Reaktionsmischung mit 200 ml Essigsäureethylester versetzt und mit gesättigter NaCl-Lösung und Wasser gewaschen. Nach Trocknen über $MgSO_4$ und Einengen der Lösung im Wasserstrahlvakuum wurde das Rohprodukt durch Flashchromatographie (Petrolether/Essigsäureethylester 5:3) gereinigt.

Die Isopropyliden-Schutzgruppen wurden durch 5 tägiges Rühren des erhaltenen Produktes in 120 ml 70 gew.-%iger Essigsäure bei Raumtemperatur entfernt. Nach Abdestillieren von Essigsäure und Wasser im Wasserstrahlvakuum wurde das Endprodukt durch Flashchromatographie (Dichlormethan/Methanol/Wasser 14:6:1) gereinigt. Die Titelverbindung wurde in einer Ausbeute von 55 % erhalten.

Beispiel 7

2-Dodecyloxy-1,3-di-(β-D-mannofuranosyloxy)-propan

Ausgehend von 2-Dodecyloxy-1,3-di(trifluormethansulfonyloxy)-propan wurde die Titelverbindung in

einer Ausbeute von 58 % analog zu Beispiel 6 hergestellt.

Beispiel 8

2-Hexadecyloxy-1,3-di-(β-D-mannofuranosyloxy)-propan

Ausgehend von 2-Hexadecyloxy-1,3-di-(trifluormethansulfonyloxy)-propan wurde die Titelverbindung in einer Ausbeute von 59 % analog zu Beispiel 6 hergestellt.

Anwendungstechnische Eigenschaften

Die Bisglykoside aus den Beispielen 1 bis 5 ($X=CH$, $R^2=R^3=H$, $n=m=1$, $p=q=0$, Gly = Glycosyl) wurden auf ihre kritische Micellbildungskonzentration (CMC) und ihre Oberflächenspannung untersucht. Als Vergleichsbeispiele wurden die entsprechenden Werte für Decyl- und Dodecyl-β-D-glucosid aus Tab. 2 der Literaturstelle (1) herangezogen.

Die CMC-Werte wurden bei 20 °C nach der üblichen Methode bestimmt.

Die Oberflächenspannung wurde gemäß DIN 53 914 gemessen. Dabei wird die Kraft in mN/m gemessen, welche notwendig ist, um einen horizontal aufgehängten Ring oder Bügel aus der Flüssigkeitsoberfläche herauszuziehen.

Die folgende Tabelle zeigt Ergebnisse der Messungen:

| Bisglykosid | $R^1$ | CMC [mmol/l] | Oberflächenspannung [mN/m] |
|---|---|---|---|
| aus Bsp. 1 | n-Decyl | 0,594 | 42,3 |
| aus Bsp. 2 | n-Dodecyl | 0,065 | 38,9 |
| aus Bsp. 3 | n-Tetradecyl | 0,0026 | 38,5 |
| aus Bsp. 4 | n-Hexadecyl | 0,0619 | 44,1 |
| aus Bsp. 5 | n-Decyloxy | 0,472 | 46,8 |
| z. Vergleich: | | | |
| Decyl-β-D-glucosid | | 1,9 | 30,5 |
| Dodecyl-β-D-glucosid | | 0,08 | 36 |

## Patentansprüche

1. Bisglykoside der allgemeinen Formel I

   $$\text{Gly-O-CHR}^2\text{-(CH}_2)_p\text{-(XR}^1)_n\text{-(CH}_2)_q\text{-(CHR}^3)_m\text{-O-Gly} \qquad \text{I}$$

   in der die Variablen folgende Bedeutung haben:

   | | |
   |---|---|
   | X | $C\text{-}R^4$ oder N |
   | $R^1$ | für den Fall $X = C\text{-}R^4$: $C_1$-$C_{30}$-Alkyl oder $C_2$-$C_{30}$-Alkenyl oder O-$C_1$-$C_{30}$-Alkyl oder O-$C_2$-$C_{30}$-Alkenyl<br>für den Fall X = N: CO-$C_1$-$C_{30}$-Alkyl oder CO-$C_2$-$C_{30}$-Alkenyl |
   | $R^2$ bis $R^4$ | Wasserstoff oder $C_1$-$C_{30}$-Alkyl oder $C_2$-$G_{30}$-Alkenyl |
   | n | 1 bis 3 |
   | m | 0 oder 1 |
   | p,q | 0 bis 2 |
   | Gly | aus Aldopentosen, Aldohexosen und/oder Ketohexosen aufgebaute Mono- oder Disaccharide. |

2. Bisglykoside I nach Anspruch 1, bei denen die den Resten $R^1$ bis $R^4$ zugrundeliegende Alkyl- oder Alkenylgruppen aus 6 bis 22 C-Atomen bestehen.

**3.** Bisglykoside der allgemeinen Formel Ia nach Anspruch 1 oder 2

$Gly\text{-}O\text{-}CHR^2\text{-}CHR^1(CHR^3)_m\text{-}O\text{-}Gly$ Ia

in der $R^1$ die für den Fall X = C-$R^4$ angegebene Bedeutung hat.

**4.** Bisglykoside der allgemeinen Formel Ib nach Anspruch 1 oder 2

$Gly\text{-}O\text{-}CHR^2\text{-}(CH_2)_p\text{-}NR^1(CH_2)_q\text{-}CHR^3\text{-}O\text{-}Gly$ Ib

in der $R^1$ die für den Fall X = N angegebene Bedeutung hat.

**5.** Verfahren zur Herstellung von Bisglykosiden I gemäß den Ansprüchen 1 bis 4, dadurch gekennzeichnet, daß man
(a) Diole der allgemeinen Formel II

$HO\text{-}CHR^2\text{-}(CH_2)_p\text{-}(XR^1)_n\text{-}(CH_2)_q\text{-}(CHR^3)_m\text{-}OH$ II

mit geschützten Glykosyl-trihalogenacetimidaten der allgemeinen Formel III

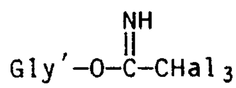

III

in der Hal für Fluor, Chlor oder Brom steht und Gly' einen Glykosylrest von Mono- oder Disacchariden, die aus Aldopentosen, Aldohexosen und/oder Ketohexosen aufgebaut sind und deren übrige Hydroxylgruppen hierbei übliche Schutzgruppen tragen, bezeichnet, oder
(b) aus den Diolen II erhältliche Bistrifluormethansulfonate der allgemeinen Formel IV

$CF_3SO_2O\text{-}CHR^2\text{-}(CH_2)_p\text{-}(XR^1)_n\text{-}(CH_2)_q\text{-}(CHR^3)_m\text{-}OSO_2CF_3$ IV

mit geschützten Mono- oder Disacchariden der allgemeinen Formel V

Gly'-OH V

umsetzt und anschließend die Schutzgruppen der Glykosylreste nach den üblichen Methoden entfernt.

**6.** Diole der allgemeinen Formel II gemäß Anspruch 5

$HO\text{-}CHR^2\text{-}(CH_2)_p\text{-}(XR^1)_n\text{-}(CH_2)_q\text{-}(CHR^3)_m\text{-}OH$ II

als Zwischenprodukt für die Herstellung der Bisglykoside I.

**7.** Verwendung von Bisglykosiden I gemäß den Ansprüchen 1 bis 4 als oberflächenaktive Substanzen und/oder als Komplexbildner und Gerüststoffe in Nasch- und Reinigungsmitteln.

**8.** Verwendung von Bisglykosiden I gemäp den Ansprüchen 1 bis 4 als Emulgatoren in Körperpflegemitteln.

**9.** Wasch-, Reinigungs- und Körperpflegemittel, enthaltend 1 bis 50 Gew.-% eines Bisglykosides I oder eines Gemisches dieser Verbindungen gemäß den Ansprüchen 1 bis 4.